# EUROPÄISCHE PATENTSCHRIFT

(11) **EP 1 442 706 B1**
(45) Veröffentlichungstag und Bekanntmachung des Hinweises auf die Patenterteilung: **13.03.2013**
(21) Anmeldenummer: 04001868.1
(22) Anmeldetag: 29.01.2004
(51) Int. Cl.: A61B 5/15

(54) **Blutentnahmevorrichtung mit einem eine Kanüle aufweisenden Halter**
Blood sampling device with a holder comprising a canula
Dispositif de prélèvement sanguin avec un support comportant une canule

(30) Priorität: 31.01.2003 DE 20301575 U
(43) Veröffentlichungstag der Anmeldung: 04.08.2004
(73) Patentinhaber: Sarstedt AG & Co., 51588 Nümbrecht (DE)
(72) Erfinder: Sarstedt, Walter, 41488 Nümbrecht (DE); Färber, Horst, 51588 Nümbrecht (DE)
(74) Vertreter: Grosse, Wolf-Dietrich Rüdiger

(56) Entgegenhaltungen:
- EP-A- 0 478 459
- WO-A-92/20281
- US-A- 3 877 465
- US-A- 4 915 702
- US-A- 5 067 490

## Beschreibung

Die Erfindung betrifft eine Blutentnahmevorrichtung enthaltend eine Führungshülseund einen darin einschraubbaren Halter, der eine mit einer angeschärften Schneidkante ausgebildete, hohlnadelartige Kanüle aufweist, die von einem endseitig geschlossenen, elastischen, schlauchartigen, durch Formschluss gehaltenen Ventilgummi umhüllt ist, wie durch die WO 92/20281 A1 bekannt geworden.

Aus der US-A 3 585 984 ist eine Blutentnahmevorrichtung mit einem Halter bekannt, der eine mit einer angeschärften Schneidkante ausgebildete, hohlnadelartige Kanüle auweist. Die Kanüle ist von einem endseitig geschlossenen, elastischen, schlauchartigen Ventilgummi mit Luft zwischen der Kanüle und dem Ventilgummi umhüllt. Der Halter besitzt nach vorne hin einen domartigen Ansatz, in den ein mit dem Ventilgummi einstückiges, verdicktes Kopfstück bzw. ein Stopfen eintaucht. Dieses Kopfstück stellt damit einen das Ventilgummi von dessen Außenmantel her festlegenden Abschnitt da. Das Ventilgummlt wird nämlich in einem vorderen Bereich, d.h. mit seinem verdickten Kopfstück formschlüssig auf die Kanüle aufgeschoben und wird demzufolge durch Reibungskräfte in seinem Inneren bezüglich der Kanüle und auf seinem Äußeren bezüglich des Halters festgelegt.

Durch die DE-C 196 17 000 ist die Befestigung eines Ventilgummis derart bekanntgeworden, daß ein das Ventilgummi von seiner Außenseite her festlegender Abschnitt des Halters als ein verformbares Klemmelement ausgebildet ist.

Bei einer durch die DE-C 18 12 742 bekanntgewordenen Blutentnahmevorrichtung wird ein einseitig offener, röhrenförmiger Halter verwendet, der eine an ihren beiden Enden mit einer angeschärften Schneidkante ausgebildete, hohlnadelartige Doppelkanüle aufweist, deren hinteres Ende in den auch als Führungshülse dienenden Halter hineinragt. Während das hintere Kanülenende der doppelschneidigen Hohlnadel von dem elastischen Ventilgummi mit radialer Luft umschlossen wird, ragt das vordere Kanülenende aus der Stirnwand des Halters vor und dient zur Einführung in eine Vene eines Patienten. Das entnommene Blut gelangt in ein Blutentnahmeröhchen, das zuvor von dem offenen Halterende her an den Halter angeschlossen worden ist. Beim Zusammenfügen von Halter und Blutentnahmeröhrchen durchdringt die Schneidkante des hinteren Kanülenendes zunächst die ihm zugewandte Wand bzw. den Boden des elastischen Ventilgummis und danach einen elastischen Verschlußstopfen des Blutentnahmeröhrchens, so daß letztendlich das hintere Kanülenende in das Blutentnahmeröhrchen hineinragt; das elastische Ventilgummi wird dabei ziehharmonikaartig zusammengedrückt. Nach der Blutentnahme und dem Abziehen des Blutentnahmeröhrchens von dem Halter nimmt das Ventilgummi selbsttätig wieder seine das hintere Kanülenende dichtend abschließende Ausgangslage ein. Der Halter könnte alternativ auch auf lediglich eine die Kanüle aufweisende Scheibe reduziert werden, d.h. die Führungshülse braucht nicht vorhanden zu sein. Die Halterscheibe ließe sich in einem solchen Fall mit dem Dom einer Kappe des Blutentnahmeröhrchens verbinden.

Eine andere Ausführung einer während der Blutentnahme mit einer Doppelkanüle ausgebildeten Blutentnahmevorrichtung ist durch das DE-U 80 16 927 bekanntgeworden. Die Doppelkanüle besteht dort aus einem einseitig offenen, zylindrischen Gehäuse mit einem einteilig angeformten Außenkonus und einer darin angeordneten einschneidigen, in situ das hintere Kanülenende bildenden Kanüle. Auf den Außenkonus wird ein Aufnahmekonus eines Halters mit einer endseitig angeschärften Kanüle bzw. Hohlnadel aufgesteckt, so daß nach dem Zusammenfügen dieser beiden Bauteile eine Doppelkanüle vorliegt, wie oben schon beschrieben. Mit dieser Nadel-Ausführung ist eine Adaption möglich, die es erlaubt, eine handelsübliche Luer-Kanüle mit einem Blutentnahmeröhrchen zu verbinden. Das auch hier vorhandene elastische Ventilgummi liegt der Hohlnadel des Halters bzw. Adapters eng an. Es liegt im Rahmen der Anwendungsmöglichkeiten, einen solchen Adapter mit Luer-Konus über einen speziellen Anschluß an einen z.B. mit Blut gefüllten Schlauch anzukoppeln.

Bei einer aus der DE-A 29 03 167 bekannten Sicherheits-Kanüle zur Mehrfach-Blutentnahme ist das elastische, sich beim Verbinden mit dem Blutentnahmeröhrchen ziehharmonikaartig zusammenschiebende Ventilgummi wiederum mit radialer Luft bzw. radialem Spiel zu dem hinteren Kanülenende der Doppelkanüle angeordnet, d.h. das Ventilgummi besitzt über seine gesamte Länge einen Außendurchmesser, der größer als der Außendurchmesser der Kanüle bzw. Hohlnadel ist.

Das bei den Blutentnahmevorrichtungen das hintere Kanülenende umschließende bzw. umhüllende Ventilgummi ermöglicht es, mehrere Blutentnahmeröhrchen durch Ankoppeln an die Doppelkanüle zu füllen. Bei einem eng an der Hohlnadel anliegenden Ventilgummi (vgl. DE-U 80 16 927) tritt zwischen der Nadel und dem Ventilgummi unvermeidlich eine Reibung auf, was dazu führt, daß das Ventilgummi aus der Ziehharmonika-Haltung zu langsam in die entspannte Ausgangsposition zurückschnellt; beim Wechseln der Blutentnahmeröhrchen ist in diesen Fällen daher häufig nicht zu vermeiden, daß Bluttropfen aus der Kanüle hervorquellen. Dies deshalb, weil das Ventilgummi das hintere Ende der angeschärften Doppelkanüle nicht schnell genug verschließen kann. Das wird dann vermieden, wenn das elastische, schlauchartige Ventilgummi einen radialen Abstand zu der Nadel einnimmt, d.h. diese mit Luft umhüllt (vgl. DE-A 29 03 167 und DE-C 18 12 742). Dadurch, daß das Ventilgummi nicht eng an der Nadel anliegt, ergeben sich allerdings Probleme, das Ventilgummi lagesicher in einem Halter bzw. Adapter vorzusehen. Das Ventilgummi der aus der DE-C 18 12 742 bekannten Blutentnahmevorrichtung ist deshalb an seinem offenen Ende mit einem Anschlagbund ausgebildet, der sich gegen eine Innenschulter des Halters anlegt und im übrigen in dieser Lage allein aufgrund der Reibung zwischen der Mantelfläche des Bundes und der Innenmantelfläche des Halters positioniert wird. Das Ventilgummi der durch die DE-A 29 03 167 bekanntgewordenen Doppelkanüle ist an seinem offenen hinteren Ende mit einem Außenbund ausgebildet, der in eine Ringnut der Stirnwand des Halters eingesetzt ist. Abgesehen davon, daß auch hier wiederum das Ventilgummi nur mit einem losen Sitz in dem Halter befestigt ist, bereitet zudem die Montage des Ventilgummis Schwierigkeiten und erfordert großes Geschick beim Einfädeln des elastischen Ventilgummis in die Ringnut.

Daneben sind zahlreiche andere Befestigungstechniken für das Ventligummi bekannt, wie gemäß der EP-A 0 678 279 das Ventilgummi mit einem umlaufenden Kragen in einen umlaufenden Rand im Inneren des Halters einzuhängen und durch einen Plastikeinsatz zu sichern. Weiterhin z.B. das formschlüssige Aufschieben des Ventilgummis auf einen zylindrischen Abschnitt im Mittelbereich der Kanüle (vgl. DE-A 37 40 269). Durch die EP-A 0 619 096 ist es bekanntgeworden, das Ventilgummi in seinem vorderen Bereich über eine kugelförmige Randwulst zu schieben, während demgegenüber die WO-A 95/16395 lehrt, das Ventilgummi über einen umlaufenden konusförmigen Bund mit Hinterschneidungen zu stülpen und dort gegebenenfalls noch zu verkleben. Bei der Befestigung des Ventilgummis auf einem solchen Pilz besteht die Gefahr, daß das Gummi bei aggressiven Sterilisationsprozessen im Bereich der Dehnung des Gummis am Pilz versprödet und hierdurch seine Dichteigenschaften verliert. Außerdem ist die Montage des Gummis auf dem Pilz schwierig und die Herstellung des Pilzes fertigungstechnisch aufwendig, da sogenannte Backenformen eingesetzt werden müssen.

Der Erfindung liegt die Aufgabe zugrunde, eine Blutentnahmevorrichtung der eingangs genannten Art mit einer verbesserten und einfacheren Halterung des Ventilgummis zu schaffen.

Diese Aufgabe wird erfindungsgemäß mit den im kennzeichnenden Teil des unabhängigen Anspruchs angegebenen Merkmalen gelöst. Hierdurch läßt sich nicht nur der Herstellungs- und Montageaufwand vereinfachen, da die Befestigung des Ventilgummis beim Zusammenfügen von Führungshülse und Kanülenhalter ohne weiteres selbsttätig erreicht wird, sondern auch eine Verwendung aggressiver Sterilisationsmedien (z.B. Ozon) ermöglichen. Denn die Befestigung des Ventilgummis erfordert keine nennenswerte Druckbelastung, die ansonsten durch die thermische Belastung des nachgeschalteten Sterilisationsvorgangs eine Rückverformung (Memory-Effekt) bewirken könnte, so daß dann keine ausreichende Haltekraft mehr gewährleistet wäre.

Die form- und kraftschlüssige Befestigung bzw. Halterung des Ventilgummis wird einerseits dadurch erreicht, daß das Ventilgummi mit einem an seinem offenen Ende ausgebildeten Bund In die Führungshülse eingehängt und durch den anschließend eingeschraubten Kanülenhalter angedrückt wird. Andererseits dadurch, dass das Ventilgummi an seinem freien Ende mit einem äußeren und einem inneren Konus ausgebildet ist. Der innere Konus sitzt hierbei mit geringstmöglicher oder sogar ohne Vorspannung auf einem konischen Ansatz am Kanülenhalter. Durch den äußeren Konus am Ventilgummi und durch eine dazu ebenfalls konische Innenkontur in einem komplementären Abschnitt der Führungshülse wird dann das Ventilgummi bei Zusammenschrauben von Kanülenhalter und Führungshülse festgelegt.

Weitere Merkmale und Einzelheiten der Erfindung ergeben sich aus der nachfolgenden Beschreibung von In den Zeichnungen dargestellten Aueführungsbeisplalen der Erfindung. Es zeigen:
- Fig.1: als Einzelheit einer Blutentnahmevorrichtung im Längsschnitt eine Führungshülse mit eingeschraubtem Kanülenhalter; und
- Fig. 2: in einer Darstellung wie zuvor eine andere Ausführung der Führungshülse mit eingeschraubtem Kanülenhalter.

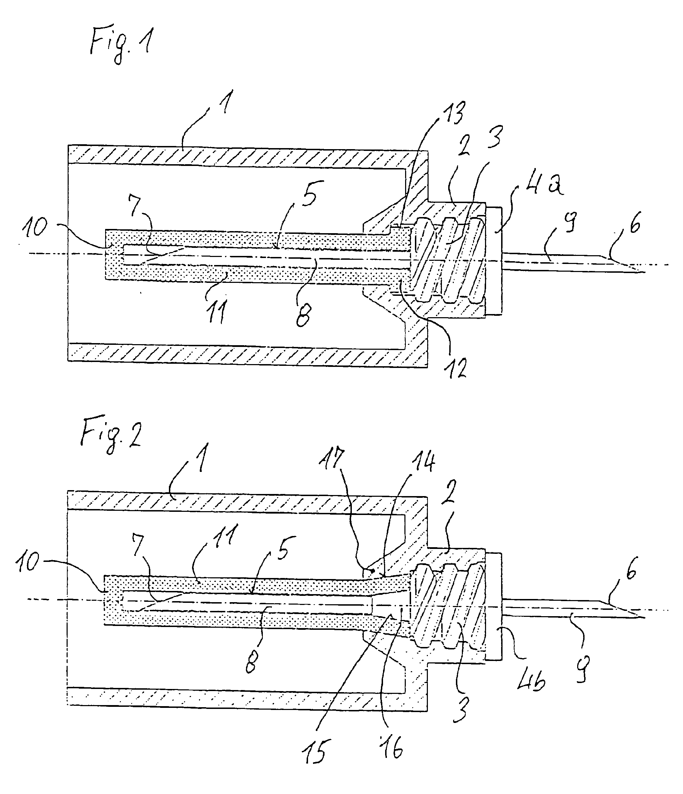

Von einer als solche hinlänglich bekannten Blutentnahmevorichtung ist in den Figuren 1 und 2 eine einseitig offene Führungshülse 1 dargestellt, die zum Gebrauch auf ein nicht gezeigtes Blutentnahmeröhrchen aufgesetzt wird. In einen Ringkragen 2 der Führungshülse 1 ist mit einem Gewindeabschnitt 3 ein Kanülenhalter 4a bzw. 4b eingeschraubt, der hier mit einer Doppelkanüle (Nadel) 5 versehen ist. Die Doppelkanüle 5 ist vorne und hinten mit einer Schneidkante 6 bzw. 7 vorsehen. Das hintere Kanülenende 8 ragt in die Führungshülse 1 hinein, während das vordere Kanülenende 9 zur Blutentnahme in eine Vene eines Patienten eingeführt wird.

Zur Blutentnahme wird die Führungshülse 1 in bekannter Weise auf ein entsprechendes Blutentnahmeröhrchen aufgeschoben, wobei die Schneidkante 7 des hinteren Kanülenendes 8 sowohl den Boden 10 eines das hintere Kanülenende 8 umhülenden Ventilgummis 11 als auch einen elastischen Verschlußstopfen des nicht dargestellten Blutentnahmeröhrchens durchdringt, bis die Verbindung zum Inneren des Blutentnahmeröhrchens hergestellt wird

Bei der Ausführung nach Fig. 1 ist das elastische, schlauchartige Ventilgummi 11 an seinem von dem Boden 10 abgewandten offenen Ende mit einem Bund 12 ausgebildet und damit in einen Kammerraum 13 der Führungshülse 1 eingehängt. Das Kopfende des Gewindeabschnittes 3 des eingeschraubten Kanülenhalters 4a drückt den Bund 12 axial an den gegenüberliegenden Grund des Kammerraumes 13 an, so daß das Ventilgummi 11 in dem Kammerraum 13 der Führungshülse 1 form- und kraftschlüssig festgelegt ist.

Das gleiche Befestigungsprinzip wird bei der Variante nach Fig. 2 dadurch verwirklicht, daß das Ventilgummi 11 im Bereich seines offenen Endes mit einem äußeren Konus 14 und einem inneren Konus 15 ausgebildet ist. Der innere Konus 15 sitzt auf einem komplementären, als Fortsatz des Gewindeabschnitts 3 des Kanülenhalters 4b vorgesehenen Kegel 16, während der äußere Konus 14 von einem innen mit einer entsprechenden konischen Kontur ausgebildeten Ansatz 17 der Führungshülse 1 umschlossen wird. Auch hier legt der axial andrückend wirkende Gewindeabschnitt 3 des Kanülenhalters 4b das Ventilgummi 11 in der Führungshülse 1 fest.

## Patentansprüche

1. Blutentnahmevorrichtung, enthaltend eine Führungshülse (1) und einen darIn einschraubbaren Halter (4a; 4b), der eine mit einer angeschärften Schneidkante (6, 7) ausgebildete, hohlnadelartige Kanüle (6) aufweist, die von einem endseitig geschlossenen, elastischen, schlauchartigen, durch Formschluss gehaltenen Ventilgummi (11) umhüllt ist,
**dadurch gekennzeichnet,**
**dass** das Ventilgummi (11) form- und kraftschlüssig in der Führungshülse (1) gehalten ist, wobei das Kopfende des Gewindeabschnitts (3) des eingeschraubten Kanülenhalters (4a) axial andrückend auf einen Bund (12) des Ventilgummis (11) wirkt und den Bund (12) an einen Grund eines Kammerraums (13) des Kanülenhalters (4a) drückt oder wobei der axial andrückend wirkende Gewindeabschnitt (3) des eingeschraubten Kanülenhalters (4b) ein Ventilgummi (11), das an seinem freien Ende mit einem äußeren Konus (14) und einem Inneren Konus (15) ausgebildet ist, wobei der Innere Konus (15) auf einem Kegel (16) als Fortsatz des Gewindeabschnitts (3) des Kanülenhalters (4b) sitzt, mit den äußerten Konus (14) an einen konischen Ansatz (17) der Führungshülse (1) festlegt.

## Claims

1. A blood withdrawal device, containing a guide sleeve (1) and a holder (4a; 4b) able to be screwed therein, which has a cannula (5) in the manner of a hollow needle, constructed with a sharpened cutting edge (6, 7), which is surrounded by an elastic, tube-like valve rubber (11) held by form fitting and closed on the end side,
**characterized in that**
the valve rubber (11) is held in a form-fitting and force-fitting manner in the guide sleeve (1), wherein the head end of the threaded section (3) of the screwed-in cannula holder (4a) acts in an axially pressing manner onto a collar (12) of the valve rubber (11) and presses the collar (12) onto a base of a chamber space (13) of the cannula holder (4a) or wherein the threaded section (3) of the screwed-in cannula holder (4b), acting in an axially pressing manner, secures a valve rubber (11), which is constructed at its free end with an outer taper (14) and an inner taper (15), wherein the inner taper (15) sits on a conical formation (16) as extension of the threaded section (3) of the cannula holder (4b), with the outer taper (14) onto a conical projection (17) of the guide sleeve (1).

## Revendications

1. Dispositif de prélèvement sanguin, comprenant un manchon de guidage (1) et un manche (4a ; 4b) pouvant être vissé dedans qui présente une canule (5) de type aiguille creuse formée avec une arête de coupe aiguisée (6, 7) qui est entourée par une valve-caoutchouc (11) fermée aux extrémités, élastique, de type tuyau et maintenue par complémentarité de forme,
**caractérisé en ce que**
la valve-caoutchouc (11) est maintenue dans le manchon de guidage (1) par complémentarité de forme et adhérence des forces, sachant que l'extrémité de tête du tronçon de filetage (3) du manche de canule (4a) vissé agit en appuyant axialement sur un épaulement (12) de la valve-caoutchouc (11) et appuie l'épaulement (12) sur un fond d'un espace de chambre (13) du manche de canule (4a) ou sachant que le tronçon de filetage (3) du manche de canule (4b) vissé agissant en appuyant axialement définit une valve-caoutchouc (11) qui est formée sur son extrémité libre d'un cône extérieur (14) et d' un cône intérieur (15), sachant que le cône intérieur (15) repose sur un cône (16) en tant que prolongement du tronçon de filetage (3) du manche de canule (4b), avec le cône extérieur (14) sur une butée conique (17) du manchon de guidage (1).
